# EUROPEAN PATENT SPECIFICATION

(11) **EP 4 133 117 B1**
(45) Date of publication and mention of the grant of the patent: **06.09.2023**
(21) Application number: 21716213.0
(22) Date of filing: 06.04.2021
(51) Int. Cl.: C22C 38/02, C22C 38/04, C22C 38/16, A61L 31/02

(54) **BIORESORBABLE FE-MN-SI-X ALLOYS FOR MEDICAL IMPLANTS**
BIORESORBIERBARE FE-MN-SI-X-LEGIERUNGEN FÜR MEDIZINISCHE IMPLANTATE
ALLIAGES FE-MN-SI-X BIORÉSORBABLES POUR IMPLANTS MÉDICAUX

(30) Priority: 10.04.2020 IT 202000007717
(43) Date of publication of application: 15.02.2023
(73) Proprietor: Saes Getters S.p.A., 20045 Lainate (MI) (IT)
(72) Inventor: CODA, Alberto, 21040 Cislago VA (IT); LEMKE, Jannis, 20148 Milano MI (IT); TUISSI, Ausonio, 23900 Lecco LC (IT); FIOCCHI, Jacopo, 23900 Lecco LC (IT); BIFFI, Carlo Alberto, 23900 Lecco LC (IT)
(74) Representative: Concone, Emanuele
(86) International application number: PCT/EP2021/058957
(87) International publication number: WO 2021/204811

(56) References cited:
- WO-A1-2014/011803
- WO-A1-2017/188908
- WO-A2-2013/059715
- US-A1- 2010 217 370

## Description

The present invention relates to metal alloy compositions, exhibiting an adequate and controlled biodegradation rate with excellent mechanical properties, suitable to be used for manufacturing bioresorbable medical implants.

Versatile biodegradable metals for short-term medical implants have been intensively studied in recent years and used as a structure (or scaffold) to support blood vessels, hollow organs and duct systems (endovascular implants), for fastening and temporary fixation of tissue implants and tissue transplants, and for orthopedic purposes such as nails, plates or screws. Some of the possible alloys for such applications, especially in the orthopedic field (bone fixation screws, pins, plates, disks), are based on iron.

Some implants are attached to reinforce damaged bones in the body, and since they are generally much stiffer than the replaced tissue, implants can promote stress shielding in the bone to which they are attached thus leading to implant loosening and poor remodeling. While surgical implant replacement is possible, replacement surgery is detrimental for the well-being of patients and usually is not performed more than once for a particular implant device due to the extent of bone damage created by the first implant. Furthermore, younger people who are still growing and get injured in accidents could particularly benefit from bioresorbable implants that will last only for several months up to years, whereas permanent implants will not fit in size anymore over time.

Implants made of bioresorbable material are designed to degrade and disappear in an application relevant time-scale. Over time, to achieve their desired purpose, the implant gradually decomposes while maintaining its structural function as long as necessary and the stresses are gradually transferred to the healing tissue.

As used herein, "biocorrodible," "bioabsorbable" and "bioresorbable" all refer to materials that can degrade and be absorbed safely within the body, thus are considered as alternative to secondary surgeries.

One approach to avoid a further surgical intervention consists in making the implant either entirely or partially of a bioresorbable metallic material. For the purposes of the present disclosure, "biodegradable" refers to processes which involve the presence of biological media and lead to a gradual degradation of the structure made of this material. Over time, the implant, or at least the part of the implant made of the bioresorbable material, loses its mechanical integrity. The degradation products are largely absorbed by the body, and small quantities of non-degradable alloy constituents are tolerable, as long as they are non-toxic.

To date, several alloys have been investigated in bioresorbable alloy development, including absorbable metals such as magnesium, zinc and iron that are expected to corrode gradually in-vivo by an appropriate host response and then dissolve completely upon assisting tissue healing. Mg- and Zn-based alloys are less suited for several orthopedic applications because of their relatively poor mechanical properties, and they may exhibit too high degradation rates or cause detrimental degradation effects, like hydrogen evolution in case of Mg alloys. On the other hand, stainless steels and Ti-based alloys possess good mechanical properties, but are not intrinsically degradable, which means an additional surgical procedure must be performed for their removal.

Generally, an adequate Young modulus (as close as possible to the replacing tissue), high yield strength (higher than 300 MPa) and a high ductility (above 18% deformation prior to failure) are targeted in the development of several implant materials. Moreover, ductility is also considered as an important characteristic in the processing from base material to implant with a desired shape. The most used metals for biomedical applications and implants, as stainless steel 316L, show moderate yield strength (i.e. comprised in the 150-250 MPa range) and a rather high Young modulus (~190 GPa), while pure Fe components exhibit a yield strength of about 150 MPa and a Young modulus of ~200 GPa in annealed conditions.

Other important features in selecting a metallic material for biomedical applications are its biocompatibility and corrosion behavior (which has a significant impact on the biocompatibility) in the biological environment of application.

Iron-based biodegradable materials are considered to be a promising alternative to permanent metallic implants and they can be designed to have attractive mechanical properties, which make them suitable for orthopedic implants. However, we have to consider that, due to the formation of a complex iron phosphate layer in body environment, the degradation rate of iron is significantly reduced, thus the disadvantage of very slow degradation in physiological pH has to be overcome. From this perspective, one of the possible routes is to add Mn to pure Fe or Fe-based alloys to moderately accelerate the corrosion and consequently improve the degradation rate.

Magnesium implants appear highly biocompatible in biological environments, and in orthopedic applications selected magnesium alloys would reduce implant-associated stress shielding and bone degradation, for example magnesium-based screws have been used with good results in bone healing clinical trials.

The main drawbacks of magnesium and its alloys are their relatively low strength and ductility, and the release of oxides, hydroxides, and hydrogen gas as byproducts during their corrosion in a physiological environment. In particular, the corrosion rate is considered particularly limiting for implant applications which have to bear mechanical loads, especially in orthopedic implants, as failure could occur before the healing process is completed.

Zinc alloys, which are also considered for biodegradable vascular and bones implants, can corrode faster than Fe-based alloys, but there is controversy on the allowed daily amount of Zn to which the human body can be exposed without health risks. Furthermore, the metallic properties of Zn, even though they can be improved to some extent by alloying elements, remain inferior to Fe-based alloys.

Among the several solutions that have been disclosed in order to provide bioresorbable alloys for medical implants, WO 2013/059715 describes a stent comprising an iron-based alloy Fe-X-Y, wherein X is at least one austenite-stabilizing element selected from the group consisting of Co, Ni, Mn, Cu, Re, Rh, Ru, Ir, Pt, Pd, C and N, and wherein Y is at least one corrosion-activator species selected between Au and Pd. In an embodiment, the stent comprises an alloy consisting essentially of 64.5 wt% Fe, 35 wt% Mn, and 0.5 wt% Pd.

EP 2676685 discloses a stent which is composed entirely or in part of an iron alloy, wherein the content of Mn is 3.0-20.0 wt%, and iron and production-related impurities make up the remainder of the 100 wt%: Mo is 0-3.5 wt%, Si is 0-3.0 wt%, Al is 0-3.0 wt%, Cu is 0-3.0 wt%. Other alloy concentrations will also be useful in alternative invention embodiments. As an example, some other stent embodiments are composed entirely or in part of an iron alloy having the composition as outlined above, where the concentrations of one or more of, and in some embodiments each of, Ni, Co, Mn, Ti, Nb, V, Mo, Si, Al and Cu are at least 0.05 wt%.

US 2010/0217370 discloses a bioerodible stent having a composition comprising Fe, Mn, Si and C, which has desirable mechanical, erosion and physiological characteristics. In another aspect, the invention features a stent body formed of an alloy consisting essentially of Fe for about 90% or more, Mn about 6% or less, and Si and/or C.

A bioabsorbable wire material including manganese (Mn) and iron (Fe) is described in WO 2014/011803, where one or more additional constituent materials (X) are added to control corrosion in an in vivo environment and, in particular, to prevent and/or substantially reduce the potential for pitting corrosion. The (X) element in the Fe-Mn-X system may include nitrogen (N), molybdenum (Mo) or chromium (Cr), or a combination of these. This promotes controlled degradation of the wire material. The alloy made in accordance with this disclosure may comprise in one embodiment at least 45 wt% iron (Fe), at least 15 wt% manganese (Mn), and an anti-corrosive alloying element comprising at least one of: between 0.05 wt% and 1.3 wt% chromium (Cr), and between 0.10 wt% and 5.0 wt.% molybdenum (Mo). In another embodiment, the invention provides a bimetal composite device comprising two biodegradable metallic materials. One of the first and second biodegradable metallic materials comprises a Fe-Mn-X alloy wherein iron (Fe) is at least 61 wt%, manganese (Mn) is at least 31 wt% and an additional alloying element (X) comprises at least one of: chromium (Cr) in an amount between 0.05 wt% and 1.3 wt%, molybdenum (Mo) in an amount between 0.10 wt% and 5.0 wt%, and nitrogen (N) in an amount between 0.01 wt% and 0.45 wt%, and the other of the first and second biodegradable metallic materials comprises a second material different from the Fe-Mn-X alloy.

WO 2017/188908 describes a tubular metal alloy stent, wherein a Fe-Mn-X-Y alloy is used for producing biodegradable stents. Fe is characterized as 47-75 wt%, Mn is 20-35 wt%, X contains at least one element selected from the group that is composed of Si, Co and Mo, and Y contains at least one element selected between C and Pd.

Iron-based solutions of the prior art have several limits, consisting in some cases in alloys having poor mechanical properties with limited elongation to failure, some of them might exhibit ferromagnetic behavior or contain elements with poor biocompatibility like Ni or Co. Furthermore, most of these alloys generally have only a slight corrosion tendency under the usual application conditions.

Purpose of this invention is to disclose new iron-manganese-silicon-based alloys that can show suitable degradation characteristics and mechanical properties at the same time, in particular for some types of medical implants, with regard to orthopedic implants. To prepare the alloys with a sufficiently high corrosion rate and increase their strength, additional elements have been added to Fe-Mn alloys, such as Si and Cu in a proper amount suitable to obtain good corrosion rates without jeopardizing their ductility.

In particular, the purpose of the present invention is to improve the performance of prior art biodegradable alloys for implant applications, which are exposed to significant mechanical loads and corrosion. The present alloys offer particular mechanical properties including a high product of strength and ductility (also referred to by the ECO-index: ultimate tensile strength x elongation to failure), a low Young modulus compared to several iron alloys (e.g. 316L, pure Fe) and homogeneous corrosion behavior. The addition of an appropriate quantity of certain alloying elements leads to an increase in strength and to acceleration of the corrosion rate, more convenient for bioresorbable implants than those of the majority of metallic alloys from the prior art. The combination of these properties allows for engineering implant devices with particular reference to orthopedic implants and degradable fracture fixation implants for hard tissues, which degrade in application-required time periods.

The Fe-Mn-Si-X alloys object of the present invention contain iron, manganese and silicon as main components and are characterized by the presence of an austenitic/martensitic matrix phase in an either monophasic or biphasic microstructure (ε, γ).

The Mn content comprised between 25-35 wt% should be ideally selected in such a way to tailor the ratio of austenite to martensite phase at room temperature which can transform into ε to obtain a good compromise between strength and ductility, and the presence of the two phases further seems to accelerate the corrosion. Alloys may contain also a few brittle precipitates (especially at high Si content) and several MnS inclusions.

The role of silicon could be diversified in the alloys object of the present invention, since it promotes the martensitic transformation with an improvement of mechanical properties and possibly induces short range ordering of the atoms to improve the reversibility of this transformation, thereby improving manufacturability. It hardens the parent matrix, through solid solution strengthening and the formation of Si-rich precipitates to suppress the dislocation gliding, and keeps the stacking-fault energy low.

The bioresorbable metal alloy composition Fe-Mn-Si-X according to the present invention comprises one or more elements X, acting in their main role as strengthening and corrosion impacting elements by affecting the precipitation behavior and the matrix electrochemical potential without significantly reducing the biocompatibility. According to the present invention, X is at least an element selected in the group consisting of copper, gold, silver, platinum and combinations thereof.

A homogenous distribution of the secondary phases/precipitates present in the matrix is important to avoid an extensive segregation of them at the grain boundaries which can cause embrittlement of the alloys.

Furthermore, the possibility to cause/prevent the presence of precipitates in adequate amount and size by the alloy preparation methods can be exploited to accelerate/decelerate the corrosion rate. Therefore, elements which form coherent precipitates, such as copper or gold, can bring several advantages, like promoting and/or increasing the corrosion speed and further improve hardness. Finally, considering the field of application of the alloys herein disclosed, said X elements can be chosen in order to bring additional features as for example Cu which has also antimicrobial properties.

Fe and Mn weight percentages are comprised respectively between 65-75 wt% and between 25-35 wt%, while the amount of Si used to obtain improved mechanical properties is comprised between 0.5-6 wt%.

The bioresorbable metal alloy composition according to the present invention contains X in an amount between 0.5 wt%, preferably higher than 1 wt%, and 3 wt%, preferably lower than 2 wt%. Depending on the selection of alloying elements X, a range of elemental ratios exists leading to a definite functional performance of the alloy. Best results for a Fe-Mn-Si-Cu alloy were obtained when silicon is in an amount preferably comprised between 4 and 6 wt% and copper is in an amount preferably comprised between 1 and 3 wt%.

The above defined ratios and amounts of elements in weight percentages of these alloys can be engineered by thermal/thermo-mechanical treatments to modify the microstructural features, including reinforcing by particle precipitation the matrix phase at room temperature and the herewith related phase transformations.

It was found that these alloys exhibit a good compromise of mechanical properties as required in orthopedic applications:
- decent elongation up to failure, specifically higher than 15%;
- adequate Young modulus (E), specifically comprised between 90 and 160 GPa, such that final medical devices can be designed with an effective Young modulus close to the replacing tissue, showing functional stability and favoring healing while avoiding stress shielding effects;
- sufficient high yield strength and ultimate tensile strength, compared to mechanical properties of reference materials for implants, targeted mechanical properties for orthopedic applications being at least higher than 300 MPa for yield strength (YS) and 700 MPa for ultimate tensile strength (UTS).

In this regard, the Fe-Mn-Si-X based alloys according to the present invention provide excellent values, and the best results are achieved selecting Cu as X element. The combined use of Si and Cu leads to elevated mechanical properties as these elements allow for the formation of two different types of controllable precipitates in the matrix of the microstructure. The presence of both elements guarantees a combination of high strength and ductility, unlike other alloying approaches using typical strengthening elements that cause, already in small quantities, embrittlement at the grain boundaries (e.g. Si + C).

In a further aspect of the invention, an advantage is related to obtaining a controlled degradation rate through high Mn content and the alloying of appropriate percentages and ratios of the X elements, and through their combination in the alloys. When considering the degradation rates, they should be slow enough to guarantee the mechanical integrity of the implant over the healing process, and fast enough to ensure that it would not cause any damage related to an extended stay in the body.

A medical implant fabricated in accordance with the alloy compositions described in the invention will degrade and preferably dissolve completely within an adequate time frame.

According to the invention, by corrosion tests it was found that Fe-Mn-Si-X alloys have fast corrosion rates compared to pure iron as well as several other Fe-Mn-Si-based alloys of the prior art.

These Fe-Mn-Si-X alloys show, in corrosion tests performed according to ASTM G5 - 94 R99, corrosion rates intermediate between pure Fe and Mg alloys, specifically comprised between 0.10 and 0.50 mm/y, and they can be processed into various absorbable medical implants. Non-limiting examples of medical devices in which the compositions and articles of the invention can be used may include screws, nails, meshes, plates, fasteners, rods, pins, wire structures, anchor clips, plugs, bone-fracture healing devices, joint replacement devices, with particular reference to bone implants.

The bioresorbable metal alloy compositions of the invention can be prepared using various methods and processes, such as by melting the pure elements, preferably in powder or pieces, in order to obtain the desired atomic ratios and concentrations. The melting must be carried out in a controlled atmosphere, for example under vacuum or inert gas, in order to avoid oxidation of the alloy which is being prepared and to keep under control the high vapor pressure of some elements. Among the most common melting technologies, but not limited to them, arc melting, vacuum induction melting (VIM), vacuum arc remelting (VAR), induction skull melting (ISM), electro slug remelting (ESR) or electron beam melting (EBM) can be used.

Further, a set of processing steps executed according to this invention is related to heat treatments. In its broadest sense, the term heat treatment refers to any of the heating and cooling operations that are performed for the purpose of changing the mechanical properties, the microstructure, or the residual stress state of a metal product.

For example, solution treatment in combination with quenching is performed to solubilize secondary phases and to freeze the material in a desired microstructural state, e.g. a certain quantity of ε/γ-phase that forms at higher temperatures. Homogenization/normalizing is used prior to hot working processes and is performed to obtain a homogenous microstructure of medium grain size. Precipitation treatments are performed to induce homogenously coherent precipitates in a prior solution-treated material, and stress release treatments are applied to relax the material without causing abundant precipitation.

A further set of processing steps executed according to this invention is related to main deformation processes and the ways in which they are used to form metallic objects of various shape or geometry. Forming processes include stamping, rolling, extrusion and forging, where deformation is induced by external compressive forces or stresses exceeding the yield stress of the material, and forming can be divided principally into the two categories of hot working and cold working. At hot working temperatures, a metal remains ductile through dynamic reforming of its grain structure, so repeated, large deformations are possible. The strain rates of many metal-working processes are so high that there is insufficient time for the metal to recrystallize as it deforms.

Cold working, which is inherent to processes that are performed at room temperature (or up to about 200°C for some metals), leads to anisotropy and increased stiffness and strength in a metal, with a corresponding decrease in ductility and malleability as the metal strain hardens. Advantages over hot working include a better surface finish, closer dimensional control of the final article and improved mechanical properties.

The invention will be further illustrated by means of the following non-limiting examples that are intended to teach the skilled person how to put the invention into practice.

### EXAMPLE 1

The experimental tests are carried out to provide information about mechanical properties of bioresorbable Fe-Mn-Si-based metal alloys. Samples S1, S2, S3, S4 and S5 represent alloy compositions according to the present invention and tested after cold rolling and heat treatment at 500-800°C for 1 hour. Comparative samples C1, C2, C3, C4, C5 and C6 show general mechanical properties for alloys and compositions not in accordance with the present invention.

C1 is a ternary Fe-Mn-Si alloy, without the addition of a precipitate-forming element X, that was hot forged at 1000°C. The chemical composition of C2 is mainly characterized by magnesium (90.1 wt%), aluminum (9.5 wt%), zinc (0.2 wt%) and manganese (0.2 wt%). C3 is standard 316L stainless steel, C4 is a binary Fe-Mn alloy and C5 is a Fe-Mn-Si-C alloy containing 69.7 wt% Fe, 25 wt% Mn, 5 wt% Si and 0.3 wt% C, which were both produced in the same way as S1. C6 is an arc-melted and then suction cast Fe-Mn-Si-Pd alloy, containing 63 wt% Fe, 30 wt% Mn, 6 wt% Si and 1 wt% Pd tested in compression.

The tests concerning the measures of elongation, Young modulus (E), yield strength (YS) and ultimate tensile strength (UTS) in order to evaluate mechanical properties of the alloys according to the invention are summarized in table A. The results reveal that Fe-Mn-Si alloys with addition of Cu, Pt and Au according to the invention have values demonstrating excellent mechanical properties in relation to the comparative samples.

In fact, it can be seen that, while S1-S6 samples display simultaneously elongation, E, Y and UTS values comprised in the above-disclosed ranges suitable for the application in the orthopedic alloys field, comparative examples C1-C6 present at least one feature which made the respective composition less suitable for said applications.

For instance, samples C1 and C2 have an insufficient elongation value, i.e. lower than 15%, while samples C1, C3 and C4 show Young modulus (E) values too high for the purpose of the application (i.e. higher than 160 GPa), and samples C2, C3 and C4 present too low values of UTS (i.e. lower than 700 MPa). Moreover, sample C5 with the use of C as X element is brittle and not possible to test, while sample C6 reported from the paper "Novel Fe-Mn-Si-Pd alloys: insights into mechanical, magnetic, corrosion resistance and biocompatibility performances", J Mater Chem B. 2016 Oct 21;4(39), clearly shows how the selection of Pd as X element in the reported alloy compositions leads to a mechanical performance characterized by a too low Young modulus (E) value and, at the same time, also a too low yield strength (YS) value with respect to the claimed compositions. For sample C6, it is noted that the following tables A and B contain only the data available from the above-mentioned paper.

**Table A**

| | Alloy | X | Composition [wt%] | | | | Condition | Elongation (%) | E (GPa) | YS (MPa) | UTS (MPa) |
|---|---|---|---|---|---|---|---|---|---|---|---|
| | | | Fe | Mn | Si | X | | | | | |
| S1 | FeMnSiX | Cu | 65 | 28 | 5 | 2 | CR+600°C x 1h | 18 | 145 | 691 | 1176 |
| S2 | FeMnSiX | Cu | 65 | 28 | 5 | 2 | CR+800°C x 1h | 38 | 149 | 481 | 966 |
| S3 | FeMnSiX | Cu | 65 | 27 | 5 | 3 | CR+600°C x 1h | 15 | 150 | 671 | 1061 |
| S4 | FeMnSiX | Cu | 69 | 27 | 2 | 2 | CR+600°C x 1h | 42 | 137 | 386 | 756 |
| S5 | FeMnSiX | Pt | 67 | 27 | 5 | 1 | CR+600°C x 1h | 22 | 110 | 332 | 956 |
| S6 | FeMnSiX | Au | 67 | 27 | 5 | 1 | CR+600°C x 1h | 16 | 108 | 346 | 937 |
| C1 | FeMnSi | - | 68 | 28 | 4 | n.a. | Hot forged at 1000°C | 14 | 164 | 450-550 | 848 |
| C2 | AZ91 | - | - | 0.2 | - | η. a. | η. a. | 7 | 45 | 160 | 250 |
| C3 | 316L | - | 62-69 | 2.0 | 0.75 | η. a. | η. a. | 60 | 193 | 205 | 515 |
| C4 | FeMn | - | 71 | 29 | - | η. a. | CR+600°C x 1h | 27 | 190 | 480 | 650 |
| C5 | FeMnSiX | C | 69.7 | 25 | 5 | 0.3 | CR+600°C x 1h | Brittle | | | |
| C6 | FeMnSiX | Pd | 63 | 30 | 6 | 1 | Suction Cast | n.a. | 60 | 270 | n.a. |

### EXAMPLE 2

The corrosion properties have been evaluated by potentiostatic and potentiodynamic tests in the presence of oxygen according to ASTM G 5 - 94 R99 in order to measure relevant corrosion parameters, like the corrosion potential (Ecorr) and corrosion rate (mm/y). Samples S1, S2, S3, S4 represent FeMnSiCu alloys, S5 and S6 FeMnSiPt and FeMnSiAu alloys according to the present invention in homogenized state, respectively. Comparative sample C1 represents a forged, ternary Fe-Mn-Si alloy, which was forged at 1000°C, such a hot forging process should not affect significantly the corrosion properties and if so, at best increase the corrosion rate a little by the introduction of defects. C4 is the binary FeMn alloy homogenized in the same way as S3, and C7 and C8 represent pure Fe and Mg respectively. Comparative examples C2 and C3 are not reported in the list as they consist of an excessively fast dissolving Mg alloy (corrosion rates >> 0.5 mm/y) and a corrosion-resistant steel (corrosion rates< < 0.01 mm/y) in such testing conditions. C5 was brittle and it was impossible to extract a sample for testing.

Results are summarized in Table B, showing that the corrosion potential and corrosion rate values of S1 - S6 demonstrate a good metal corrosion compared to a pure Fe and pure Mg. Comparative examples C1, C4 and C6 show corrosion potential and corrosion rate values similar to those of S1-S6, however it's confirmed that they are not suitable for the application in the orthopedic alloys field due to the mechanical properties described above.

**Table B**

| | **Alloy** | **Ecorr (V)** | **Corr. Rate (mm/y)** |
|---|---|---|---|
| S1/S2 | FeMnSiCu | -0.83 | 0.30 |
| S3 | FeMnSiCu | -0.84 | 0.33 |
| S4 | FeMnSiCu | -0.79 | 0.22 |
| S5 | FeMnSiPt | -0.81 | 0.14 |
| S6 | FeMnSiAu | -0.83 | 0.19 |
| C1 | FeMnSi | -0.82 | 0.25 |
| C4 | FeMn | -0.82 | 0.29 |
| C6 | FeMnSiPd | -0.77 | n.a. |
| C7 | Pure Fe | -0.38 | 0.01 |
| C8 | Pure Mg | -1.62 | 1.5-2 |

## Claims

1. A bioresorbable metal alloy composition comprising Fe-Mn-Si-X, wherein the Fe content is comprised between 65 wt% and 75 wt%, the Mn content is comprised between 25 wt% and 35 wt%, and the Si content is comprised between 0.5 wt% and 6 wt%, **characterized in that** X is at least an element selected in the group consisting of copper, gold, silver, platinum and combinations thereof and **in that** the X content is comprised between 0.5 wt% and 3 wt%.

2. Bioresorbable metal alloy composition according to claim 1, wherein the Fe content is comprised between 67 wt% and 72 wt%.

3. Bioresorbable metal alloy composition according to claim 1 or 2, wherein the Mn content is comprised between 27 wt% and 30 wt%.

4. Bioresorbable metal alloy composition according to any of claims 1 to 3, wherein the Si content is comprised between 4 wt% and 6 wt%.

5. Bioresorbable metal alloy composition according to any of claims 1 to 4, wherein the X content is comprised between 1 wt% and 2 wt%.

6. Bioresorbable metal alloy composition according to any of claims 1 to 4, wherein X is copper in an amount comprised between 1 wt% and 3 wt%.

7. A bioresorbable medical implant comprising a bioresorbable metal alloy composition according to any of the preceding claims.

8. Bioresorbable medical implant according to claim 7, comprising one or more apparatuses selected in the group consisting of screws, nails, meshes, plates, fasteners, rods, pins, wire structures, anchor clips, plugs, bone-fracture healing devices and joint replacement devices.

## Patentansprüche

1. Bioresorbierbare Metalllegierungszusammensetzung, umfassend Fe-Mn-Si-X, wobei der Fe-Gehalt zwischen 65 Gew.-% und 75 Gew.-%, der Mn-Gehalt zwischen 25 Gew.-% und 35 Gew.-% und der Si-Gehalt zwischen 0,5 Gew.-% und 6 Gew.-% liegt,
**dadurch gekennzeichnet, dass**
X mindestens ein Element ist, das aus der Gruppe aus Kupfer, Gold, Silber, Platin und Kombinationen davon ausgewählt ist, und dass der X-Gehalt zwischen 0,5 Gew.-% und 3 Gew.-% liegt.

2. Bioresorbierbare Metalllegierungszusammensetzung nach Anspruch 1,
wobei der Fe-Gehalt zwischen 67 Gew.-% und 72 Gew.-% liegt.

3. Bioresorbierbare Metalllegierungszusammensetzung nach Anspruch 1 oder 2, wobei der Mn-Gehalt zwischen 27 Gew.-% und 30 Gew.-% liegt.

4. Bioresorbierbare Metalllegierungszusammensetzung nach einem der Ansprüche 1 bis 3,
wobei der Si-Gehalt zwischen 4 Gew.-% und 6 Gew.-% liegt.

5. Bioresorbierbare Metalllegierungszusammensetzung nach einem der Ansprüche 1 bis 4,
wobei der X-Gehalt zwischen 1 Gew.-% und 2 Gew.-% liegt.

6. Bioresorbierbare Metalllegierungszusammensetzung nach einem der Ansprüche 1 bis 4,
wobei X Kupfer in einer Menge zwischen 1 Gew.-% und 3 Gew.-% ist.

7. Bioresorbierbares medizinisches Implantat, umfassend eine bioresorbierbare Metalllegierungszusammensetzung nach einem der vorangehenden Ansprüche.

8. Bioresorbierbares medizinisches Implantat nach Anspruch 7, umfassend eine oder mehrere Vorrichtungen, die aus der Gruppe umfassend Schrauben, Nägel, Maschen, Platten, Befestigungselemente, Stäbe, Stifte, Drahtstrukturen, Verankerungsclips, Stöpsel, Vorrichtungen zur Heilung von Knochenbrüchen und Gelenkersatzvorrichtungen ausgewählt sind.

## Revendications

1. Composition d'alliage métallique biorésorbable comprenant Fe-Mn-Si-X, dans laquelle la teneur en Fe est comprise entre 65 % en poids et 75 % en poids, la teneur en Mn est comprise entre 25 % en poids et 35 % en poids, et la teneur en Si est comprise entre 0,5 % en poids et 6 % en poids, **caractérisée en ce que** X est au moins un élément choisi dans le groupe constitué par le cuivre, l'or, l'argent, le platine et leurs combinaisons, et **en ce que** la teneur en X est comprise entre 0,5 % en poids et 3 % en poids.

2. Composition d'alliage métallique biorésorbable selon la revendication 1, dans laquelle la teneur en Fe est comprise entre 67 % en poids et 72 % en poids.

3. Composition d'alliage métallique biorésorbable selon la revendication 1 ou 2, dans laquelle la teneur en Mn est comprise entre 27 % en poids et 30 % en poids.

4. Composition d'alliage métallique biorésorbable selon l'une quelconque des revendications 1 à 3, dans laquelle la teneur en Si est comprise entre 4 % en poids et 6 % en poids.

5. Composition d'alliage métallique biorésorbable selon l'une quelconque des revendications 1 à 4, dans laquelle la teneur en X est comprise entre 1 % en poids et 2 % en poids.

6. Composition d'alliage métallique biorésorbable selon l'une quelconque des revendications 1 à 4, dans laquelle X est le cuivre en une quantité comprise entre 1 % en poids et 3 % en poids.

7. Implant médical biorésorbable comprenant une composition d'alliage métallique biorésorbable selon l'une quelconque des revendications précédentes.

8. Implant médical biorésorbable selon la revendication 7, comprenant un ou plusieurs dispositifs choisis dans le groupe constitué par les vis, les clous, les filets, les plaques, les dispositifs de fixation, les tiges, les broches, les structures maillées, les pinces d'ancrage, les fiches, les dispositifs de cicatrisation de fractures osseuses, et les dispositifs de prothèse articulaire.
